# EUROPÄISCHE PATENTSCHRIFT

(11) **EP 4 303 567 B1**
(45) Veröffentlichungstag und Bekanntmachung des Hinweises auf die Patenterteilung: **21.08.2024**
(21) Anmeldenummer: 22182896.5
(22) Anmeldetag: 04.07.2022
(51) Int. Cl.: G01N 21/359, G01N 33/46, G01N 21/47

(54) **VERFAHREN ZUR BESTIMMUNG DER MENGE VON MINDESTENS EINEM PULVERFÖRMIGEN BINDEMITTEL IN EINER MISCHUNG MIT HOLZPARTIKELN**
METHOD FOR DETERMINING THE VOLUME OF AT LEAST ONE POWDERY BINDER IN A MIXTURE WITH WOOD PARTICLES
PROCÉDÉ DE DÉTERMINATION DE LA QUANTITÉ D'AU MOINS UN LIANT PULVÉRULENT DANS UN MÉLANGE COMPORTANT DES PARTICULES DE BOIS

(43) Veröffentlichungstag der Anmeldung: 10.01.2024
(73) Patentinhaber: Flooring Technologies Ltd., Kalkara SCM1001 (MT)
(72) Erfinder: Kalwa, Norbert, 32805 Horn-Bad Meinberg (DE); Zhang, Jinming, 10247 Berlin (DE)
(74) Vertreter: Maikowski & Ninnemann Patentanwälte Partnerschaft mbB

(56) Entgegenhaltungen:
- WO-A1-97/04299
- US-A1- 2007 131 862
- US-B1- 6 414 312

## Beschreibung

Die vorliegende Erfindung betrifft ein Verfahren zur Bestimmung bzw. Messung der Menge von mindestens einem biologisch abbaubaren pulverförmigen Bindemittel in einer Mischung mit Holzpartikeln und ein Verfahren zur Herstellung von Holzwerkstoffplatten aus dieser Mischung aus pulverförmigen Bindemittel und Holzpartikeln unter Verwendung dieses Messverfahrens.

### Beschreibung

Holzwerkstoffplatten, wie Holzspanplatten oder Holzfaserplatten, wobei vorliegend unter Holzfaserplatten immer mittel- oder hochdichte Holzfaserplatten (MDF/HDF) zu verstehen sind, bilden die Basis vieler Gegenstände des täglichen Lebens, beispielsweise von Möbeln oder Belägen für Wand, Boden oder Decke. OSB-Platten (oriented strand boards) werden im Holz- und Fertighausbau eingesetzt, da OSB-Platten leicht sind und trotzdem die an Bauplatten gesetzten statischen Anforderungen erfüllen. So werden OSB-Platten als Bauplatten und als Wand- oder Dachbeplankung oder auch im Fußbodenbereich eingesetzt. Die angeführten Holzwerkstoffplatten werden in mehrstufigen Prozessen hergestellt, wobei am Anfang dieser Prozesse jeweils ein Schritt des Vermischens der entsprechenden Holzpartikel mit eine geeigneten Bindemittel steht, gefolgt vom Streuen des Gemisches auf ein Transportband und Verpressen des aufgestreuten Gemisches zu einer Holzpartikelmatte bzw.- platte. Die typischerweise verwendeten Bindemittel bzw. Leime basieren meist auf Harnstoff-Formaldehyd-, Phenol-Formaldehyd- oder PMDI-Leimen (Polymeres Diphenylmethandiisocyanat ).

Auch auf die Holzwerkstoffindustrie kommt mehr und mehr die Forderung zu, diese erdölbasierten Komponenten durch nachwachsende Rohstoffe zu substituieren. Beim Einsatz von Leimen auf Basis von nachwachsenden Rohstoffen stehen aktuell eine Vielzahl von Produkten zur Auswahl. Zur Vermeidung einer Konkurrenzsituation zwischen der Nutzung als Nahrungsmittel und der Verwendung als Leim, werden mehr und mehr ohnehin anfallende Reste aus der Produktion von Lebensmitteln eingesetzt.

Auch Reststoffe aus anderen industriellen Produktionen kommen zum Einsatz. Diese stammen dann z. B. aus der Herstellung von Zellstoff ( Lignin) oder aus Kulturpflanzenabfällen (Rapstrester usw.). Es kommen aber auch noch Produkte, die als Nahrungsmittel genutzt werden zum Einsatz (Zucker, Stärke, Sojamehl). Bei Lignin handelt es sich um ein Makromolekül auf Basis phenolischer Komponenten. Sojamehl und Rapstrester enthalten als Hauptkomponenten Proteine und Öle. Zucker und Stärke sind Kohlenhydrate.

Diese Leimalternativen fallen als pulverförmige Produkte an und besitzen mit Ausnahme von Zucker keine gute Wasserlöslichkeit. Sie könnten, wenn die vorhandene technische Ausstattung genutzt werden soll, nur als Dispersion in der Produktion eingesetzt werden.

Die oben beschriebenen bisher eingesetzten erdölbasierten Leime sind aber alle Flüssigkeiten, die sich durch Sprühsysteme in Mischer oder Beleimtrommeln homogen auf die Holzpartikel oder Fasern dosieren lassen. Diese Dosierung ist bereits seit Jahrzehnten in die Produktion eingeführt und wurde z. B. durch Düsenoptimierungen oder durch Hochdruck-Beleimungen bezüglich der Verteilung des Leimes und der benötigten Menge weiter optimiert.

Für die Leime auf Basis von nachwachsenden Rohstoffen sind Dispersionen eher ungünstig, da Dispersionen relativ viel Wasser in das System eintragen, was für den Prozess problematisch ist. Zu viel Wasser im Span- oder Faserkuchen kann zu Dampfspaltern führen, was durch ein Nachtrocknen der Späne/Faser vermieden werden müsste.

Aus diesen Gründen kommen als Alternative eher Dosierungen als Pulver in Frage. Die erfolgt über Dosierschnecken, die das Pulver auf die Holzpartikel/-fasern auftragen. Eine weitere Verteilung kann dann in z. B. in Mischern erfolgen.

Dabei kann die Verteilung auf der Holzmatrix allerdings je nach Pulver sehr unterschiedlich sein. Dabei ist unklar, ob die vorhandenen Mischer überhaupt eine homogene Verteilung ermöglichen. Eine Beurteilung der Verteilung der Leimpartikel auf den Holzpartikeln kann allenfalls visuell erfolgen. Allerdings liegen die Korngrössen der Pulver in dem Bereich von 1 bis 200 µm, was diese Beurteilung erschwert. Auch haben die Pulver teilweise einen ähnlichen Farbton wie die Holzpartikel, was ebenfalls eine objektive Beurteilung der Pulververteilung erschwert. Auch ist weder eine kontinuierliche noch eine in-line Beurteilung in der Produktion möglich. Dies bedeutet, dass, um gewisse Mindestwerte bei der Festigkeit und der Quellung zu erreichen, eine Höherdosierung des Leimpulvers vorgenommen werden muss. Dies führt zu unnötigem Rohstoffverbrauch und höheren Kosten.

Eine mögliche Lösung für dieses Problem wäre eine Einfärbung des Pulvers oder der Einsatz einer UV-aktiven Substanz, welche dann mit Hilfe einer speziellen Lampe eine Beurteilung der Verteilung zuließe. Aber diese würde zunächst eine homogene Verteilung des Farbstoffes oder der UV-aktiven Verbindung im Pulver erfordern und würde zusätzliche Kosten erzeugen. Zudem ist die Beurteilung, ob eine Veränderung der Dosierung eine bessere Verteilung erbringt, nur am Produkt durch Bestimmung der technologischen Werte möglich. Die kann zu Fehlproduktionen/ Ausschuss führen.

Ein Beispiel für ein Verfahren zur Bestimmung der Menge eines pulverförmigen Bindemittels in einer Mischung mit Holzpartikeln und mit Hilfe der NIR-Spektroskopie ist in der Patentschrift US2007/131862 A1 angegeben.

Aus den bisherigen Herstellungsverfahren ergeben sich Nachteile. So sind die Produktionslinien zur Herstellung von Holzwerkstoffplatten für Flüssigleime optimiert. Bei der Verwendung von pulverförmigen Leimen oder Bindemitteln kann es aufgrund fehlender Analytik zu einer unnötigen Höherdosierung kommen, was mit höheren Kosten verbunden ist. Zudem können unklare Produktionsumstände auftreten.

Der vorliegenden Erfindung liegt daher die technische Aufgabe zu Grunde eine Analysemethode bereitzustellen mit deren Hilfe die Pulververteilung von biologisch abbaubarem pulverförmigem Bindemittel auf den Holzpartikelr kontinuierlich in der Produktion bestimmt werden kann. Diese sollte an jeder beliebigen Stelle in der Produktion möglich sein und durch eine hohe Messfrequenz schnell eine Vielzahl von Daten liefern. Die Messungen sollten zerstörungsfrei erfolgen und keinen großen technischen Aufwand erfordern. Veränderungen an den Anlagen zur Installation der Messanalytik sollte nicht nötig sein. Die in der Produktion an den verschiedenen möglichen Messpunkten vorliegenden Bedingungen sollten die Messungen nicht stören.

Diese Aufgabe wird erfindungsgemäß durch ein Verfahren mit den Merkmalen des Anspruchs 1 gelöst.

Demnach wird ein Verfahren zur Bestimmung der Menge von mindestens einem biologisch abbaubaren pulverförmigen Bindemittel in einer Mischung mit Holzpartikeln bereitgestellt, wobei das Verfahren die folgenden Schritte umfasst:
- Bereitstellen von Mischungen aus mindestens einem pulverförmigen Bindemittel und Holzpartikeln als Referenzproben, wobei das mindestens eine pulverförmige Bindemittel und Holzpartikel in den Mischungen in jeweils quantitativ definierten Mischungsverhältnissen vorliegen,
- Aufnahme von mindestens einem NIR-Spektrum der Referenzproben unter Verwendung von mindestens einem NIR-Messkopf in einem Wellenlängenbereich zwischen 900 nm und 1700 nm (Nanometer),
- Bereitstellen einer zu vermessenden Mischung aus mindestens einem pulverförmigen Bindemittel und Holzpartikel,
- Aufnehmen von mindestens einem NIR-Spektrum der Mischung aus dem mindestens einen pulverförmigen Bindemittel mit Holzpartikel unter Verwendung des mindestens einen NIR-Messkopfes in einem Wellenlängenbereich zwischen 900 nm und 1700 nm, und
- Bestimmen des quantitativen Anteils an pulverförmigen Bindemittel in der Mischung aus pulverförmigen Bindemittel und Holzpartikel durch Vergleich mit dem für die Referenzproben aufgenommenen NIR-Spektren.

Gemäß dem vorliegenden Verfahren wird mittels NIR-Spektroskopie die Menge eines pulverförmigen Bindemittels und ggfs. Zusammensetzung einer Kombination von zwei oder mehreren pulverförmigen Bindemitteln in einer Mischung mit Holzpartikeln bestimmt, wobei das Verfahren im laufenden Fertigungsbetrieb von Holzwerkstoffplatten einsetzbar ist. Das NIR-Messverfahren kann an verschiedenen Positionen oder Prozessschritten der Holzwerkstoffplattenproduktion durchgeführt werden, wie noch weiter unten im Detail erläutert wird. So kann der der NIR-Messkopf, insbesondere NIR-Multimesskopf, nicht nur eine einzelne Messposition berücksichtigen, sondern auch traversierend über einen Span-, Strand- oder Faserkuchen geführt werden. Damit können z. B. Randeffekte, die bei der Produktion von HWS häufig auftreten erfasst werden. Es können auch vorteilhaft einzelne Schichten gemessen werden ( Deckschicht/Mittelschicht). Ein weiterer Vorteil ist, dass aus den NIR-Spektren auch weitere Parameter (z. B.: Feuchte, siehe hierzu z.B. EP 2 915 658 B1, EP 2 808636 B1) analysiert werden können. Es ist bekannt, dass Leime auf Basis nachwachsender Rohstoffe bestimmte Mindestmengen an Feuchtigkeit für die Aushärtung brauchen. Ausserdem verbessert die Feuchtigkeit auch die Haftung des Leims auf den Holzpartikeln oder -fasern. Dies kann ebenfalls helfen Qualitätsmängel zu vermeiden.

Das vorliegende Verfahren ermöglicht die Bereitstellung der Messwerte in kurzer Zeit (online, bevorzugt ohne störende Zeitverzögerung) im Vergleich zu herkömmlichen (bekannten) Messverfahren. Die Messdaten können zur Qualitätssicherung, Forschung und Entwicklung, zur Prozesskontrolle, Prozessregelung, Prozesssteuerung usw. eingesetzt werden. Durch den Messvorgang wird die Produktionsgeschwindigkeit usw. nicht reduziert. Grundsätzlich wird damit die Überwachung der Produktion verbessert. Zudem werden auch Stillstandszeiten durch Qualitätsbestimmungen und Anlagenjustierungen reduziert.

Die mit dem vorliegenden Verfahren mögliche Bestimmung der Menge an pulverförmigen Bindemittel oder Bindemittelgemisch erfolgt bevorzugt ausschließlich mittels NIR-Messung. Eine Kombination mit anderen spektroskopische Methoden, insbesondere unter Verwendung von anderen Wellenlängen außerhalb des NIR-Bereiches ist nicht vorgesehen.

Es erfolgt somit der Einsatz eines NIR-Messkopfes, bevorzugt eines NIR-Multimesskopfes, der über die Aufnahme von spektralen Daten (Spektren) in nah infrarotem Bereich (700-2000 nm) eine Bestimmung der Menge an pulverförmigen Bindemittel zulässt. Die NIR-Strahlung wechselwirkt mit den organischen Funktionsgruppen, wie zum Beispiel O-H, C-H und N-H, die in den Bindemittel vorhanden sind. Während der Wechselwirkung wird die NIR-Strahlung durch die gemessene Probe gestreut und reflektiert. Durch den Empfang der reflektierten NIR-Strahlung per NIR-Detektor wird ein NIR-Spektrum erzeugt.. Bei dieser Messung werden in einer Sekunde eine Vielzahl von einzelnen NIR-Messungen durchgeführt, sodass auch eine statistische Absicherung der Werte gewährleistet wird. Die NIR-Spektroskopie bietet eine Möglichkeit an, einen direkten Bezug zwischen den spektralen Informationen (NIR-Spektren) und den zu bestimmenden Parametern der Bindemittel herzustellen.

Das vorliegende Verfahren nutzt den Umstand aus, dass die NIR-Strahlung in einem gewissen Umfang in die oberflächennahe Bereiche des Materials eindringt, allerdings der Großteil der NIR-Strahlung an der Oberfläche einer Holzpartikelmischung oder eines Holzpartikelkuchens reflektiert bzw. gestreut wird. Die reflektierte bzw. gestreute NIR-Strahlung wird von dem NIR-Detektor erfasst, und das ermittelte NIR-Spektrum wird zur Bestimmung der gewünschten Parameter (hier Menge an pulverförmigen Bindemittel) verwendet.

Für die Bestimmung der Menge an pulverförmigen Bindemittel werden bevorzugt spektrale Daten aus dem gesamten aufgenommenen Spektralbereich verwendet, d.h. es wird nicht eine einzelne, diskrete Wellenlänge, sondern vielmehr ein ganzer Bereich von mehreren Wellenlängen verwendet.

Gemäß dem erfindungsgemäßen Verfahren werden demnach zunächst Mischungen aus mindestens einem pulverförmigen Bindemittel und Holzpartikeln als Referenzproben bereitgestellt, wobei das mindestens eine pulverförmige Bindemittel und die Holzpartikel in den Mischungen in jeweils quantitativ definierten Mischungsverhältnissen vorliegen. Für die Bereitstellung der Referenzproben werden verschiedene Mengen an pulverförmigen Bindemittel mit Holzpartikeln gemischt, z.B. 5 Gew%, 7 Gew%, 10 Gew%, 15 Gew% an pulverförmigen Bindemittel bezogen auf die Gesamtmenge der Bindemittel-Holzpartikel-Mischung.

Zu beachten ist auch, dass die Referenzprobe gleichartig zu der zu vermessenden Probe ist; d.h. insbesondere die Bindemittel-Holzpartikel-Mischung der Referenzprobe weist die gleiche Zusammensetzung wie die zu vermessende Bindemittel-Holzpartikel-Mischung auf. Die Gleichartigkeit von zu vermessender Probe und Referenzprobe ist insbesondere bei Verwendung von Zusatzstoffen wie Flammschutzmitteln, Fasern, weiteren Additiven bedeutsam.

Von diesen Referenzproben werden zumindest ein NIR-Spektrum in einem Wellenlängenbereich zwischen 700 nm und 2000 nm, bevorzugt zwischen 900 nm und 1700 nm, insbesondere bevorzugt zwischen 1400 nm und 1600 nm aufgenommen.

Die unterschiedlichen quantitativen Mengen an pulverförmigen Bindemittel der Referenzproben werden dann den jeweils aufgenommen NIR-Spektren dieser Referenzproben zugeordnet, und es wird ein Zusammenhang zwischen den spektralen Daten der NIR-Spektren der Referenzproben und den dazugehörigen Bindemittelmengen als Parameterwert erstellt, d.h. zu jedem Parameterwert der Referenzprobe korrespondiert ein NIR-Spektrum der Referenzprobe.

Anschließend wird mindestens eine Mischung aus mindestens einem pulverförmigen Bindemittel und Holzpartikel bereitgestellt, und mindestens ein NIR-Spektrum der Mischung aus dem mindestens einen pulverförmigen Bindemittel mit Holzpartikel unter Verwendung des mindestens einen NIR-Messkopfes in einem Wellenlängenbereich zwischen 700 nm und 2000 nm aufgenommen. Die quantitative Menge bzw. der Anteil des pulverförmigen Bindemittels in der Mischung aus pulverförmigen Bindemittel und Holzpartikel kann anschließend durch Vergleich mit dem für die Referenzproben aufgenommenen NIR-Spektren ermittelt werden.

Wie bereits erwähnt, erfolgt ein Vergleich und die Interpretation der NIR-Spektren sinnvollerweise über den gesamten aufgenommenen Spektralbereich. So werden in einer Ausführungsform für die Bestimmung der Menge von mindestens einem pulverförmigen Bindemittel in einer Mischung mit Holzpartikeln spektrale Daten aus dem NIR-Spektralbereich zwischen 900 nm und 1700 nm verwendet. In einer anderen Ausführungsform werden für die Bestimmung der Menge von mindestens einem pulverförmigen Bindemittel in einer Mischung mit Holzpartikeln spektrale Daten aus dem NIR-Spektralbereich zwischen 1450 nm und 1650 nm, bevorzugt zwischen 1500 nm und 1600 nm verwendet. In noch einer anderen Ausführungsform werden für die Bestimmung der Menge von mindestens einem pulverförmigen Bindemittel in einer Mischung mit Holzpartikeln spektrale Daten aus dem NIR-Spektralbereich zwischen 900 nm und 1100 nm, bevorzugt zwischen 900 nm und 1000 nm verwendet.

In einer Ausführungsform des vorliegenden Verfahrens beträgt der Mengenanteil des pulverförmigen Bindemittels in der Mischung mit den Holzpartikeln zwischen 5 und 50 Gew%, bevorzugt zwischen 7 und 40 Gew%, insbesondere bevorzugt zwischen 10 und 30 Gew%, noch mehr bevorzugt zwischen 15 und 20 Gew%. In einer bevorzugten Ausführungsform beträgt der Mengenanteil des pulverförmigen Bindemittels in der Mischung mit den Holzpartikeln 5 Gew%, 7 Gew%, 10 Gew% oder 15 Gew% (bezogen auf die Gesamtmenge der Bindemittel-Holzpartikel-Mischung).

Das vorliegende Messverfahren kann zur Bestimmung der Menge an jeglicher Art von biologisch abbaubarem pulverförmigen Bindemittel verwendet werden.

Biologisch abbaubare Bindemittel können sowohl natürlich vorkommende Bindemittel oder auch synthetische Bindemittel sein.

Im Falle der Verwendung eines natürlichen Bindemittels als biologisch abbaubares Bindemittel ist dieses ausgewählt aus einer Gruppe enthaltend Stärke, Zellulosederivate, wie Carboxymethyl-Zellulose, Chitosan, glutenhaltige Bindemittel, wie Hautleim, Knochenleim, Lederleim; milchproteinhaltige Bindemittel, insbesondere aus der Gruppe der Kaseine, und pflanzenproteinhaltige Bindemittel, insbesondere aus der Gruppe der Sojabindemittel, Agar-Agar, Alginat, Gelantine, Guar gum, Gum arabic, Xanthan Gum, Pektine, Johannisbrotkernmehl, Polysaccharide, wie Carrageen, Lignin oder Rapstrester. Die Verwendung von Stärke und Sojamehl ist besonders bevorzugt.

In einer Ausführungsform der vorliegenden Holzfasermatte ist die als Bindemittel verwendete Stärke ausgewählt aus der Gruppe enthaltend Kartoffelstärke, Maisstärke, Weizenstärke, Reisstärke.

Stärke ist ein Polysaccharid mit der Formel (C₆H₁₀O₅)ₙ, das aus α-D-Glucose-Einheiten besteht. Das Makromolekül zählt daher zu den Kohlenhydraten. Stärke kann unter Hitzeeinwirkung ein Vielfaches ihres Eigengewichtes an Wasser physikalisch binden, aufquellen und verkleistern. Beim Erhitzen mit Wasser quillt die Stärke bei 47-57 °C, die Schichten platzen, und bei 55-87 °C (Kartoffelstärke bei 62,5 °C, Weizenstärke bei 67,5 °C) entsteht Stärkekleister, welcher je nach der Stärkesorte verschiedenes Steifungsvermögen besitzt (Maisstärkekleister größeres als Weizenstärkekleister, dieser größeres als Kartoffelstärkekleister) und sich mehr oder weniger leicht unter Säuerung zersetzt.

Stärke ist sowohl in der nativen Form als auch in modifizierter (derivatisierter) Form als Bindemittel einsetzbar. So kann die mindestens eine Stärke in nativer oder modifizierter (derivatisierter) Form in der Holzfasermatte vorliegen. Bevorzugt wird als stärkehaltiges Bindemittel DuraBinders der Firma Ecosynthetix verwendet.

Im Falle der Verwendung von modifizierter bzw. derivatisierter Stärke als Bindemittel kann diese ausgewählt sein aus einer Gruppe enthaltend kationische oder anionische Stärke, carboxylierte Stärke, carboxy-methylierte Stärke, sulfatierte Stärke, phosphorylierte Stärke, veretherte Stärke wie hydroxyalkylierte Stärke (z.B. hydroxyethylierte Stärke, hydroxypropylierte Stärke), oxidierte Stärke enthaltend Carboxyl- oder Dialdehydgruppen und hydrophobe Stärken wie Acetat-, Succinat-, Halb- oder Phosphatester.

Es ist auch generell vorstellbar eine Mischung einer natürlichen Stärke und einer derivatisierten Stärke oder von mehreren natürlichen Stärken und/oder mehreren derivatisierten Stärken zu verwenden.

Die Teilchengröße der Stärke liegt zwischen 20 bis 100 µm, bevorzugt zwischen 30 und 80 µm, insbesondere bevorzugt zwischen 40 und 60 µm, z.B. bei 50 µm.

In einer Ausführungsform des vorliegenden Verfahrens werden bei Verwendung von Stärke als pulverförmigen Bindemittel für die Bestimmung der Menge der Stärke in einer Mischung mit Holzpartikeln spektrale Daten aus dem NIR-Spektralbereich zwischen 900 nm und 1100 nm, bevorzugt zwischen 900 nm und 1000 nm verwendet.

Die Teilchengröße des verwendeten Sojamehls liegt zwischen 30 bis 300 µm, bevorzugt zwischen 50 und 200 µm, insbesondere bevorzugt zwischen 60 und 100 µm, z.B. bei 70 µm.

In einer Ausführungsform des vorliegenden Verfahrens werden bei Verwendung von Sojamehl als pulverförmigen Bindemittel für die Bestimmung der Menge des Sojamehls in einer Mischung mit Holzpartikeln spektrale Daten aus dem NIR-Spektralbereich zwischen 1450 nm und 1650 nm, bevorzugt zwischen 1500 nm und 1600 nm verwendet.

Das vorliegende spektroskopische Verfahren ermöglicht ebenfalls die Bestimmung der Menge und der Zusammensetzung einer Kombination von mindestens zwei pulverförmigen Bindemitteln in einer Mischung mit Holzpartikeln. Dies ergibt sich insbesondere aus dem Umstand, dass unterschiedliche spektrale Bereiche des NIR-Spektrums zur Auswertung herangezogen werden können.

In einer solchen Kombination können z.B. ein erstes pulverförmiges Bindemittel und ein zweites pulverförmiges Bindemittel in einem Verhältnis zwischen 10 Gew%: 90 Gew% und 90 Gew% : 10 Gew%, bevorzugt zwischen 25 Gew% :75 Gew% und 75 Gew% : 25 Gew%, insbesondere bevorzugt zwischen 55 Gew%: 45 Gew% und 45 Gew%: 55 Gew%, z.B. 50 : 50 Gew% vorliegen. Eine bevorzugte Kombination kann z.B. aus Stärke und Sojamehl bestehen. Bei Verwendung von Bindemittelgemischen kann es notwendig sein, ein Kalibrationsmodell unter Verwendung einer multivariaten Datenanalyse (MDA) für die Referenzproben zu erstellen. Bei multivariaten Analysemethoden werden in an sich bekannter Weise typischerweise mehrere statistische Variablen zugleich untersucht. Hierzu wird bei diesen Methoden üblicherweise die in einem Datensatz enthaltene Zahl von Variablen reduziert, ohne gleichzeitig die darin enthaltene Information zu mindern. Im vorliegenden Fall erfolgt die multivariate Datenanalyse über das Verfahren der Partial Least Squares Regression (partielle Regression der kleinsten Quadrate, PLS) wodurch ein geeignetes Kalibrationsmodell erstellt werden kann. Die Auswertung der gewonnenen Daten wird bevorzugt mit einer geeigneten Analysesoftware vorgenommen wie z.B. mit der Analysesoftware SIMCA-P der Firma Umetrics AB oder The Unscrambler der Firma CAMO.

Im Falle der Verwendung eines synthetischen Bindemittels als biologisch abbaubares Bindemittel ist dieses bevorzugt ausgewählt aus der Gruppe enthaltend verseiften Polyvinylalkohol, Polycaprolactam-Polyamid, Polylactat, aliphatische Polyesterharze, insbesondere Polybutylensuccinat, Polybutylensuccinat-Adipat, Polyethylen-Polypropylen-Verbundharz, wobei Polylactate besonders bevorzugt sind. In einer besonders bevorzugten Ausführungsform werden Polymilchsäurefasern mit einer Länge 38 mm +/- 3 mm und einer Feinheit von 1,7 dtex verwendet.

Wie bereits oben angedeutet, werden bevorzugt Holzfasern, Holzspäne oder Holzstrands als Holzpartikel verwendet.

Holzfasern oder -späne können durch Zerspanung der Holzhackschnitzel in einem Zerspaner oder ein Zerfaserungsprozess der Holzhackschnitzel in einem Refiner gewonnen werden.

Die typischerweise zur Herstellung der Holzfaserplatten verwendeten Holzfasern, insbesondere trockene Holzfasern, weisen eine Länge von 1,5 mm bis 20 mm und eine Dicke von 0,05 mm bis 1 mm auf.

Die zur Herstellung von OSB verwendeten Holzstrands können eine Länge zwischen 50 bis 200 mm, bevorzugt 70 bis 180 mm, insbesondere bevorzugt 90 bis 150 mm; eine Breite zwischen 5 bis 50 mm, bevorzugt 10 bis 30 mm, insbesondere bevorzugt 15 bis 20 mm; und eine Dicke zwischen 0,1 und 2 mm, bevorzugt zwischen 0, 3 und 1,5 mm, insbesondere bevorzugt zwischen 0, 4 und 1 mm aufweisen.

Es ist jedoch generell auch denkbar, dass vorliegende Messverfahren auch für andere aus nachwachsenden Rohstoffen stammende Vorprodukten zur Herstellung von Platten oder Formteilen einzusetzen (z. B. Stroh, Hanf, Bagasse usw.).

Wie oben bereits angedeutet, kann das erfindungsgemäße Verfahren zur Bestimmung der Menge an pulverförmigen Bindemittel in einer Holzpartikelmischung kontinuierlich und online in einer Fertigungslinie zur Herstellung von Holzwerkstoffplatten erfolgen. Insbesondere kann das Verfahren in einem automatisch geregelten System mit Alarmmeldung durchgeführt werden. In einer Ausführungsform kann die Bestimmung der Menge von mindestens einem pulverförmigen Bindemittel in einer Mischung mit Holzpartikeln jedoch auch offline erfolgt.

Die Bestimmung der Menge von pulverförmigen Bindemittel in einer Holzpartikelmischung kann dabei an verschieden Prozessstationen in der Fertigungslinie zur Herstellung von Holzwerkstoffplatten erfolgen.

Ein Verfahren zur Herstellung von Holzwerkstoffplatten unter Verwendung eines pulverförmigen Bindemittels umfasst die folgenden Schritte:
a) Herstellen von Holzpartikeln aus geeigneten Hölzern,
b) ggf. Zwischenlagerung der Holzpartikel, insbesondere in Silos oder Bunkern,
c) Trocknen der Holzpartikel,
d) Sortieren bzw. Sichtung der Holzpartikel entsprechend der Größe der Holzpartikel,
e) Vermischen der Holzpartikel mit mindestens einem pulverförmigen Bindemittel, z.B. unter Verwendung von Dosierschnecken;
f) Aufbringen der Mischung aus Holzpartikeln und dem mindestens einem pulverförmigen Bindemittel auf ein Transportband mittels Wind- und/oder Wurfsichtung, und
g) Verpressen der auf dem Transportband angeordneten Mischung aus Holzpartikeln und dem mindestens einem pulverförmigen Bindemittel.

Die Bestimmung der Menge des mindestens einen pulverförmigen Bindemittel in der Mischung mit Holzpartikeln unter Verwendung des erfindungsgemäßen Messverfahrens kann z.B.
i) nach dem Vermischen der Holzpartikel mit dem mindestens einem pulverförmigen Bindemittel aber noch vor dem Aufbringen der Mischung aus Holzpartikeln und dem mindestens einem pulverförmigen Bindemittel auf ein Transportband (d.h. nach Schritt e), und/oder
ii) nach dem Aufbringen der Mischung aus Holzpartikeln und dem mindestens einem pulverförmigen Bindemittel auf ein Transportband mittels Wind- und/oder Wurfsichtung, aber noch vor dem Verpressen der auf dem Transportband angeordneten Mischung aus Holzpartikeln und dem mindestens einem pulverförmigen Bindemittel (d.h. nach Schritt f), und/oder
iii) nach Verpressen der auf dem Transportband angeordneten Mischung aus Holzpartikeln und dem mindestens einem pulverförmigen Bindemittel zu einem Holzpartikelkuchen (d.h. nach Schritt g) erfolgen.

Es kann auch vorteilhaft sein einzelne Schichten zu messen, wie z.B. Deckschicht/Mittelschicht bei der Herstellung von Holzspanplatten und OSB. Zudem misst NIR nicht nur in der Oberfläche, sondern dringt auch bis in oberflächennahe Bereiche ein. Dies kann besonders die Einflüsse der Streuungen ( Wind-/Wurfstreuungen ) analysieren.

insbesondere die Vermessung der auf einem Transportband abgelegten Bindemittel-Holzpartikel-Mischung und des nach Verpressen erhaltenen Holzpartikelkuchens (d.h. Span-, Strand- oder Faserkuchens) kann traversierend erfolgen. Dabei bewegt sich der mindestens eine NIR-Messkopf quer zur Laufrichtung der abgelegten oder verpressten Bindemittel-Holzpartikel-Mischung in der Fertigungslinie über die gesamte Breite der abgelegten oder verpressten Bindemittel-Holzpartikel-Mischung, um bestimmte Problembereiche, insbesondere Minderaufträge im Rand- oder Mittelbereich der abgelegten oder verpressten Bindemittel-Holzpartikel-Mischung zu analysieren. Damit können z. B. Randeffekte, die bei der Produktion von Holzwerkstoffplatten häufig auftreten erfasst werden.

Es wird somit ein Verfahren bereitgestellt, in welchem durch die Verwendung eines NIR-Messkopfes, die Menge eines pulverförmigen Bindemittels in einer Bindemittel-Holzpartikel-Mischung aus einem einzigen NIR-Spektrum bzw. der Reflektion bzw. Streuung von NIR-Strahlung bestimmt werden kann, und zwar durch eine berührungslose Messung. Die mit dem Messkopf oder den Messköpfen ermittelten Daten werden in einer vorteilhaften Ausprägung der Erfindung direkt zur Anlagensteuerung oder -regelung verwendet.

Zudem wird in einer weiteren vorteilhaften Ausprägung der Erfindung durch die Speicherung der Daten eine Verbesserung der Qualitätskontrolle ermöglicht. Auch bei Anlagenversuchen können die gespeicherten Daten vorteilhaft einen Beitrag zur Auswertung liefern, z.B. Inbetriebnahme einer Anlage bei Neuinstallation oder nach einer Wartung oder Reparatur oder zu in-situ Testzwecken von neuen Produktions- oder Messverfahren. Durch das sofortige Vorliegen der Messwerte und der hohen Messfrequenz wird eine sehr enge Kontrolle bzw. Steuerung oder Regelung der Anlagen ermöglicht.

Die Vorteile des vorliegenden Verfahrens sind vielfältig: Berührungslose Multiparameterbestimmung ("real time"- oder "Echtzeit"-Messung) mit deutlich reduzierter zeitlicher Verzögerung in der Auswertung der gemessenen Parameterwerte; verbesserte Anlagensteuerung bzw. -regelung, Verringerung des Ausschusses, Verbesserung der Qualität der auf der Anlage hergestellten Produkte, Verbesserung der Anlagenverfügbarkeit.

Das Steuerungssystem der jeweiligen Fertigungsanlage umfasst mindestens eine computergestützte Auswerteeinheit (bzw. Prozessoreinheit) und eine Datenbank. In der Auswerteeinheit erfolgt der Abgleich bzw. Vergleich des für das Produkt (d.h. beschichtetes Trägermaterial) gemessenen NIR-Spektrums mit den für die jeweils einzelnen Parameter erstellten Kalibriermodellen. Die so bestimmten Parameterdaten werden in der Datenbank gespeichert.

Die mit dem vorliegenden spektroskopischen Verfahren bestimmten Daten können zur Steuerung der jeweiligen Fertigungslinie verwendet werden. Die berührungslos gemessenen Parameterwerte des NIR-Multimesskopfes ("Ist-Werte") können, wie zuvor bereits beschrieben, direkt und in "real time" für die Steuerung bzw. Regelung der betreffenden Anlage verwendet werden, indem beispielsweise die gemessenen und in der Datenbank, z.B. einer relationalen Datenbank, Ist-Werte gespeichert und mit dort vorhandenen Soll-Werten dieser Parameter verglichen werden. Die sich ergebenden Differenzen werden anschließend zur Steuerung bzw. Regelung der Produktionslinie verwendet.

Für den Abgleich und die Steuerung der jeweiligen Fertigungslinie wird ein computerimplementiertes Verfahren sowie ein Computerprogramm umfassend Befehle, die bei der Ausführung des Programms durch einen Computer diesen veranlassen das computerimplementierte Verfahren auszuführen, bereitgestellt. Das Computerprogramm ist in einer Speichereinheit des Steuerungssystems der jeweiligen Fertigungslinie gespeichert.

Im Folgenden werden Verfahren zu Herstellung von Holzfaserplatten, Holzspanplatten und OSB, in denen das erfindungsgemäße Messverfahren einsetzbar ist, im Detail beschrieben.

Holzfaserplatten und Holzspanplatten werden üblicherweise in einem Verfahren mit den folgenden Schritten hergestellt:
a) Herstellen von Hackschnitzeln aus geeigneten Hölzern,
b) Zerspanen der Hackschnitzel zu Holzspänen oder Holzfasern,
c) Zwischenlagerung der Holzspäne oder Holzfasern, insbesondere in Silos oder Bunkern,
d) Trocknen der Holzspäne oder Holzfasern,
e) Sortieren bzw. Sichtung der Holzspäne oder Holzfasern entsprechend der Größe der Holzspäne oder Holzfasern,
f) ggf. weitere Zerkleinerung der Holzspäne oder Holzfasern und Zwischenlagerung,
g) Vermischen der Holzspäne oder Holzfasern mit mindestens einem pulverförmigen Bindemittel, z,B. unter Verwendung von Dosierschnecken;
h) Aufbringen der Mischung aus Holzspänen oder Holzfasern und dem mindestens einem pulverförmigen Bindemittel auf ein Transportband mittels Wind- und/oder Wurfsichtung, und
i) Verpressen der auf dem Transportband angeordneten Holzspäne oder Holzfasern.

Die Bestimmung der Menge des mindestens einen pulverförmigen Bindemittel in der Mischung mit Holzspänen / Holzfasern unter Verwendung des erfindungsgemäßen Messverfahrens kann
i) nach dem Vermischen der Holzspäne / Holzfasern mit dem mindestens einem pulverförmigen Bindemittel aber noch vor dem Aufbringen der Mischung aus Holzspäne / Holzfasern und dem mindestens einem pulverförmigen Bindemittel auf ein Transportband (d.h. nach Schritt g), und/oder
ii) nach dem Aufbringen der Mischung aus Holzspäne / Holzfasern und dem mindestens einem pulverförmigen Bindemittel auf ein Transportband mittels Wind- und/oder Wurfsichtung, aber noch vor dem Verpressen der auf dem Transportband angeordneten Mischung aus Holzspäne / Holzfasern und dem mindestens einem pulverförmigen Bindemittel (d.h. nach Schritt h), und/oder
iii) nach Verpressen der auf dem Transportband angeordneten Mischung aus Holzspäne / Holzfasern und dem mindestens einem pulverförmigen Bindemittel zu einem Holzpartikelkuchen (d.h. nach Schritt i) erfolgen.

Die Verfahren zur Herstellung von Spanplatten und Holzfaserplatten unterscheiden sich insbesondere in Hinblick auf die Größe und Beschaffenheit der verwendeten Holzfasern oder Holzspäne sowie in Hinblick auf die verwendeten Drücke und Temperaturen. Der wesentliche Verfahrensablauf und damit die Reihenfolge der Verfahrensschritte sind bei allen Platten jedoch ähnlich und dem Fachmann bekannt.

Im Falle von Holzfaserplatten wird die Mischung aus Holzfasen und Bindemittel auf ein Transportband zu einem einschichtigen Faserkuchen gestreut, der vor dem Heißpressen einer Vorpressung unterzogen wird. Entsprechend wäre zwischen dem Schritt des Vorpressens und des Heißpressens ebenfalls eine (zusätzliche) NIR-Messung gemäß dem erfindungsgemäßen Verfahren denkbar.

Im Falle von Holzspanplatten wird die Mischung aus Holzspänen und Bindemittel auf ein Transportband unter Ausbildung eines mehrschichtigen Spankuchens gestreut, wobei die Holzspäne übereinander als erste Deckschicht, Mittelschicht und zweite Deckschicht gestreut werden. Entsprechend wäre eine (zusätzliche) NIR-Messung gemäß dem erfindungsgemäßen Verfahren jeweils nach dem Streuen der einzelnen Schichten denkbar, d.h. nach erster Deckschicht / nach der Mittelschicht / nach der zweiten Deckschicht.

Die Herstellung der OSB-Platten erfolgt ebenfalls in einem mehrstufigen Prozess, wobei zunächst die Strands aus entrindetem Rundholz, bevorzugt Nadelhölzer, in Längsrichtung durch rotierende Messer abgeschält werden. Im sich anschließenden Trocknungsvorgang wird die natürliche Feuchtigkeit der Strands bei hohen Temperaturen reduziert. Der Feuchtigkeitsgrad der Strands kann je nach verwendeten Bindemittel variieren, wobei sich die Feuchtigkeit deutlich unter 10% bewegen sollte, um Spalter beim späteren Verpressen zu vermeiden. In Abhängigkeit vom Bindemittel kann eine Benetzung auf eher feuchten Strands oder auf trockenen Strands günstiger sein. Außerdem sollte während des Pressvorganges möglichst wenig Feuchtigkeit in den Strands vorhanden sein, um den während des Pressvorganges entstehenden Dampfdruck weitestgehend zu reduzieren, da dieser die Rohplatte ansonsten zum Platzen bringen könnte.

Im Anschluss an die Trocknung der Strands werden diese mit einem geeigneten Bindemittel vermischt. Anschließend werden die Mischung aus Strands und Bindemittel in Streuapparaturen alternierend längs und quer zur Produktionsrichtung gestreut, so dass die Strands kreuzweise in mindestens drei Schichten angeordnet sind (untere Deckschicht - Mittelschicht - obere Deckschicht). Die Streurichtung von unterer und oberer Deckschicht ist dabei gleich, weichen jedoch von der Streurichtung der Mittelschicht ab. Auch unterscheiden sich die in der Deckschicht und Mittelschicht verwendeten Strands voneinander. So sind die in den Deckschichten verwendeten Strands flächig und die in der Mittelschicht verwendeten Strands weniger flächig bis hin zu spanförmig. Üblicherweise werden bei der Herstellung der OSB-Platten zwei Materialstränge gefahren: einer mit flächigen Strands für die späteren Deckschichten und einer mit "Spänen" für die Mittelschicht. Entsprechend können die Strands in der Mittelschicht qualitativ schlechter sein, da die Biegefestigkeit im Wesentlichen durch die Deckschichten erzeugt wird. Deshalb kann auch Feingut, das beim Zerspanen entsteht, in der Mittelschicht von OSB-Platten verwendet werden.

Im Anschluss an die Streuung der Strands erfolgt ein kontinuierliches Verpressen der selbigen unter hohem Druck und hoher Temperatur von z.B. 200 bis 250°C.

Entsprechend wäre eine NIR-Messung gemäß dem erfindungsgemäßen Messverfahren jeweils nach dem Vermischen der Strands mit dem mindestens einem pulverförmigen Bindemittel, nach dem Streuen der einzelnen Schichten, d.h. nach erster Deckschicht / nach der Mittelschicht / nach der zweiten Deckschicht, und/oder nach dem Verpressen der auf ein Transportband gestreuten Mischung aus Holzstrands und Bindemittel möglich.

Die Erfindung wird nachfolgend an Ausführungsbeispielen unter Bezugnahme auf die Figuren näher erläutert. Es zeigen:
- Figur 1: NIR Spektren von Holzspänen, Sojamehl und Gemischen von Sojamehl und Holzspänen
- Figur 2: NIR Spektren von Holzspänen, Stärke und Gemischen von Stärke und Holzspänen
- Figur 3: Ausschnitt aus NIR Spektren von Holzspänen und Gemischen von Stärke und Holzspänen

### Ausführungsbeispiel 1: Mischung aus Holzspänen und Sojamehl

Deckschichtspäne für die Herstellung von Spanplatten wurden mit verschiedenen Mengen an Sojamehl (Prolia) versetzt ( 5, 7, 10 und 15 Gew% ). Das Sojamehl hatte eine mittlere Korngrösse von 70 µm ( max. Korngrösse < 200 µm ). Die Späne und es Mehl wurden mit Hilfe einer Labormühle homogen durchmischt. Danach wurden die Gemische auf einem Teller mit Hilfe eines NIR-Messkopfes analysiert. Eine Spanprobe und eine Sojamehl-Probe wurden ebenfalls vermessen.

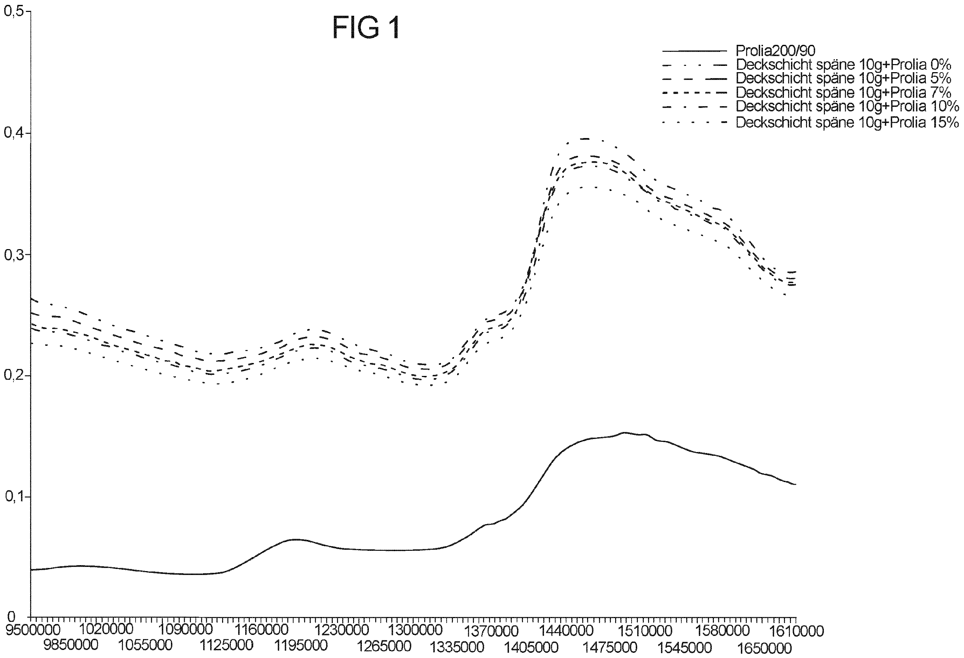

Wie sich zeigte war eine deutliche Abstufung zwischen den einzelnen Konzentrationen erkennbar. Die Kalibration/Auswertung ist am besten bei dem Peak bei ca. 1550 - 1560 nm möglich (siehe Diagramm der Figur 1).

### Ausführungsbeispiel 2: Mischung aus Holzspänen und Stärke

Deckschichtspäne für die Herstellung von Spanplatten wurden mit verschiedenen Mengen an Maisstärke versetzt (5, 10 und 15 Gew%). Die Maisstärke hatte eine mittlere Korngrösse von 50 µm (max. Korngrösse < 100 µm). Die Späne und die Stärke wurden mit Hilfe einer Labormühle homogen durchmischt. Danach wurden die Gemische auf einem Teller mit Hilfe eines NIR-Messkopfes analysiert.

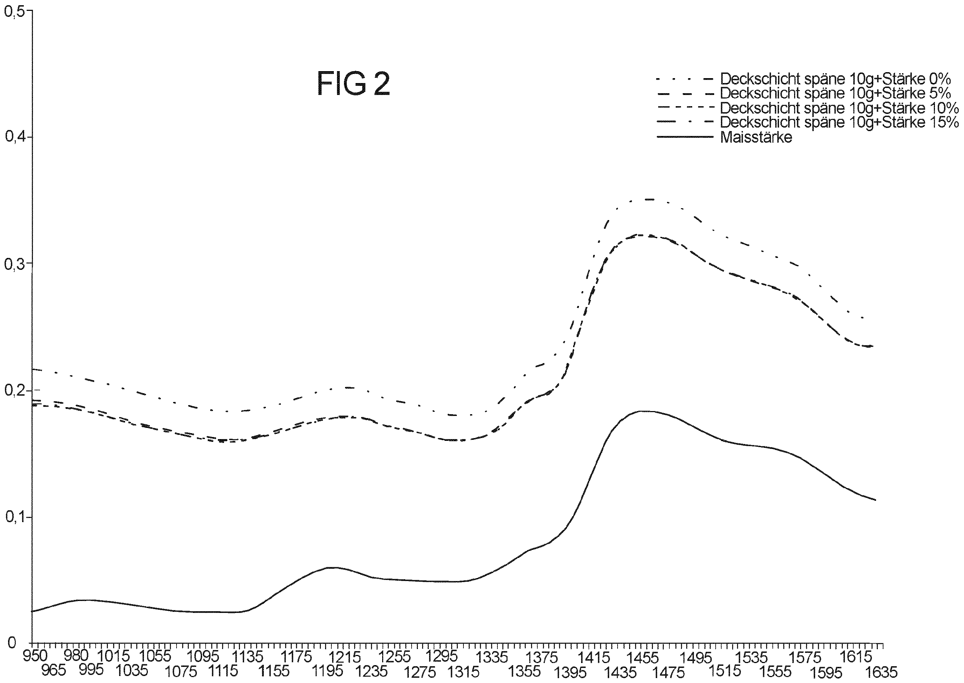

Eine Spanprobe und eine Stärkemehl-Probe wurden ebenfalls vermessen. Wie sich zeigte war in dem Bereich zwischen 1550 und 1560 nm eine deutliche schwächere Abstufung zwischen den einzelnen Konzentrationen erkennbar. Dies trifft insbesondere für 5 und 10 Gew% zu (siehe Diagramm der Figur 2)..

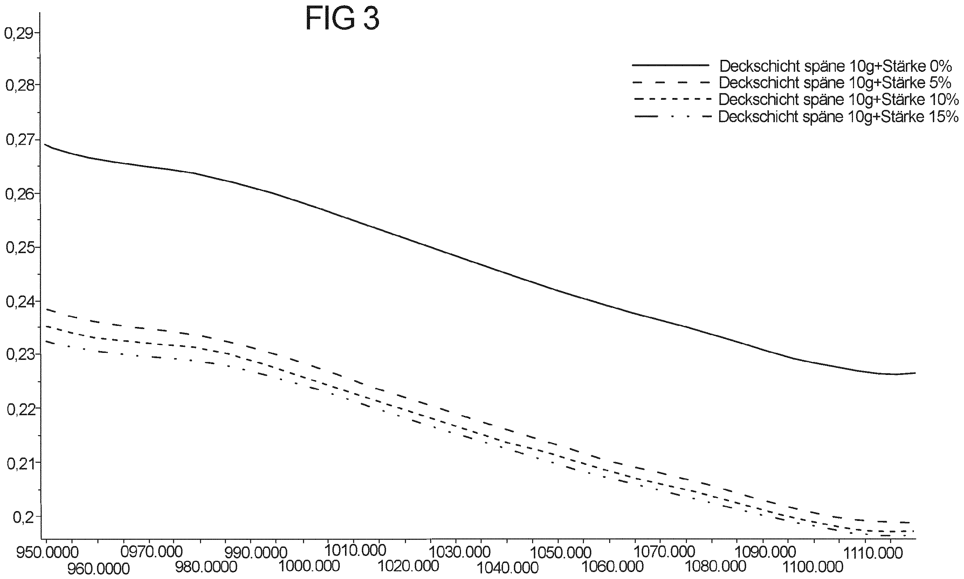

Aus diesem Grund wird für die Kalibration der Bereich zwischen 950 - 995 nm ausgewählt. Dort sind die Spektren mit steigendem Stärkegehalt getrennt (siehe Diagramm der Figur 3).

Wie sich zeigt, ist es möglich verschiedene Pulverleime sicher zu bestimmen. Die bestimmten Konzentrationen liegen dabei in den Konzentrationsbereichen, die auch bei der Herstellung von HWS zur Anwendung kommen würden. Mit Hilfe eines NIR-Messkopfes können also Konzentrationen der Biopulverleime auf partikel- oder faserförmigem Holz ebenso wie deren Schwankungen bestimmt werden. Da sich die zur Auswertung herangezogenen Peaks in unterschiedlichen Bereichen des NIR-Spektrums liegen, können auch Kombinationen aus verschiedenen Bioleimen analysiert werden.

## Patentansprüche

1. Verfahren zur Bestimmung der Menge von mindestens einem biologisch abbaubaren pulverförmigen Bindemittel in einer Mischung mit Holzpartikeln umfassend die Schritte
- Bereitstellen von Mischungen aus mindestens einem pulverförmigen Bindemittel und Holzpartikeln als Referenzproben, wobei das mindestens eine pulverförmige Bindemittel und Holzpartikel in den Mischungen in jeweils quantitativ definierten Mischungsverhältnissen vorliegen,
- Aufnahme von mindestens einem NIR-Spektrum der Referenzproben unter Verwendung von mindestens einem NIR-Messkopf in einem Wellenlängenbereich zwischen 900 nm und 1700 nm,
- Bereitstellen einer zu vermessenden Mischung aus mindestens einem pulverförmigen Bindemittel und Holzpartikel,
- Aufnehmen von mindestens einem NIR-Spektrum der Mischung aus dem mindestens einen pulverförmigen Bindemittel mit Holzpartikel unter Verwendung des mindestens einen NIR-Messkopfes in einem Wellenlängenbereich zwischen 900 nm und 1700 nm, und
- Bestimmen des quantitativen Anteils an pulverförmigen Bindemittel in der Mischung aus pulverförmigen Bindemittel und Holzpartikel durch Vergleich mit dem für die Referenzproben aufgenommenen NIR-Spektren.

2. Verfahren nach Anspruch 1, **dadurch gekennzeichnet, dass** für die Bestimmung der Menge von mindestens einem pulverförmigen Bindemittel in einer Mischung mit Holzpartikeln spektrale Daten aus dem gesamten aufgenommenen Spektralbereich verwendet werden.

3. Verfahren nach einem der vorhergehenden Ansprüche, **dadurch gekennzeichnet, dass** für die Bestimmung der der Menge von mindestens einem pulverförmigen Bindemittel in einer Mischung mit Holzpartikeln spektrale Daten aus dem NIR-Spektralbereich zwischen 900 nm und 1600 nm verwendet werden.

4. Verfahren nach einem der vorhergehenden Ansprüche, **dadurch gekennzeichnet, dass** für die Bestimmung der Menge von mindestens einem pulverförmigen Bindemittel in einer Mischung mit Holzpartikeln spektrale Daten aus dem NIR-Spektralbereich zwischen 1450 nm und 1600 nm, bevorzugt zwischen 1500 nm und 1600 nm verwendet werden.

5. Verfahren nach einem der Ansprüche 1-3, **dadurch gekennzeichnet, dass** für die Bestimmung der Menge von mindestens einem pulverförmigen Bindemittel in einer Mischung mit Holzpartikeln spektrale Daten aus dem NIR-Spektralbereich zwischen 900 nm und 1100 nm, bevorzugt zwischen 900 nm und 1000 nm verwendet werden.

6. Verfahren nach einem der vorhergehenden Ansprüche, **dadurch gekennzeichnet, dass** der Mengenanteil des pulverförmigen Bindemittels in der Mischung mit den Holzpartikeln zwischen 5 und 50 Gew%, bevorzugt zwischen 7 und 40 Gew%, insbesondere bevorzugt zwischen 10 und 30 Gew%, noch mehr bevorzugt zwischen 15 und 20 Gew% (bezogen auf die Gesamtmenge der Bindemittel-Holzpartikel-Mischung) beträgt.

7. Verfahren nach einem der vorhergehenden Ansprüche, **dadurch gekennzeichnet, dass** ein natürlich vorkommendes Bindemittel als biologisch abbaubares Bindemittel, insbesondere ausgewählt aus einer Gruppe enthaltend Stärke, Zellulosederivate, wie Carboxymethyl-Zellulose, Chitosan, glutenhaltige Bindemittel, wie Hautleim, Knochenleim, Lederleim; milchproteinhaltige Bindemittel, insbesondere aus der Gruppe der Kaseine, und pflanzenproteinhaltige Bindemittel, insbesondere aus der Gruppe der Sojabindemittel, Agar-Agar, Alginat, Gelantine, Guar gum, Gum arabic, Xanthan Gum, Stärke, Pektine, Johannisbrotkernmehl, Polysaccharide, wie Carrageen, verwendet wird.

8. Verfahren nach einem der Ansprüche 1-6, **dadurch gekennzeichnet, dass** ein synthetisches Bindemittel als biologisch abbaubares Bindemittel, insbesondere ausgewählt aus der Gruppe enthaltend verseiften Polyvinylalkohol, Polycaprolactam-Polyamid, Polylactat, aliphatische Polyesterharze, insbesondere Polybutylensuccinat, Polybutylensuccinat-Adipat, Polyethylen-Polypropylen-Verbundharz, bevorzugt Polylactate, verwendet werden.

9. Verfahren nach einem der vorhergehenden Ansprüche, **dadurch gekennzeichnet, dass** als Holzpartikel Holzspäne, Holzfasern und/oder Holzstrands verwendet werden.

10. Verfahren nach einem der vorhergehenden Ansprüche, **dadurch gekennzeichnet, dass** die Bestimmung der Menge von mindestens einem pulverförmigen Bindemittel in einer Mischung mit Holzpartikeln kontinuierlich online in einer Produktionslinie für Holzwerkstoffplatten erfolgt.

11. Verfahren nach einem der vorhergehenden Ansprüche, **dadurch gekennzeichnet, dass** die Bestimmung der Menge von mindestens einem pulverförmigen Bindemittel in einer Mischung mit Holzpartikeln offline erfolgt.

12. Verfahren zur Herstellung von Holzwerkstoffplatten umfassend:
a) Herstellen von Holzpartikeln aus geeigneten Hölzern,
b) ggf. Zwischenlagerung der Holzpartikel, insbesondere in Silos oder Bunkern,
c) Trocknen der Holzpartikel,
d) Sortieren bzw. Sichtung der Holzpartikel entsprechend der Größe der Holzstrands,
e) Vermischen der Holzpartikel mit mindestens einem biologisch abbaubaren pulverförmigen Bindemittel;
f) Aufbringen der Mischung aus Holzpartikeln und dem mindestens einem pulverförmigen Bindemittel auf ein Transportband mittels Wind- und/oder Wurfsichtung, und
g) Verpressen der auf dem Transportband angeordneten Mischung aus Holzpartikeln und dem mindestens einem pulverförmigen Bindemittel,
wobei die Bestimmung der Menge des mindestens einen pulverförmigen Bindemittel in der Mischung mit Holzpartikeln gemäß einem Verfahren nach einem der Ansprüche 1 - 11
i) nach dem Vermischen der Holzpartikel mit dem mindestens einem pulverförmigen Bindemittel aber noch vor dem Aufbringen der Mischung aus Holzpartikeln und dem mindestens einem pulverförmigen Bindemittel auf ein Transportband (nach Schritt e), und/oder
ii) nach dem Aufbringen der Mischung aus Holzpartikeln und dem mindestens einem pulverförmigen Bindemittel auf ein Transportband mittels Wind- und/oder Wurfsichtung, aber noch vor dem Verpressen der auf dem Transportband angeordneten Mischung aus Holzpartikeln und dem mindestens einem pulverförmigen Bindemittel (nach Schritt f), und/oder
iii) Nach Verpressen der auf dem Transportband angeordneten Mischung aus Holzpartikeln und dem mindestens einem pulverförmigen Bindemittel zu einem Holzpartikelkuchen (nach Schritt g) erfolgt.

## Claims

1. Method for determining the amount of at least one biodegradable powdery binder in a mixture with wood particles
comprehensive steps
- Providing mixtures of at least one powdery binder and wood particles as reference samples, wherein the at least one powdery binder and wood particles are present in the mixtures in quantitatively defined mixing ratios in each case,
- Recording of at least one NIR spectrum of the reference samples using at least one NIR measuring head in a wavelength range between 900 nm and 1700 nm,
- Providing a mixture to be measured consisting of at least one powdery binder and wood particles,
- Recording of at least one NIR spectrum of the mixture of the at least one powdery binder with wood particles using the at least one NIR measuring head in a wavelength range between 900 nm and 1700 nm, and
- Determining the quantitative amount of powdery binder in the mixture of powdery binder and wood particles by comparison with the NIR spectra recorded for the reference samples.

2. Method according to claim 1, **characterized in that** spectral data from the entire recorded spectral range are used to determine the amount of at least one powdery binder in a mixture with wood particles.

3. Method according to one of the preceding claims, **characterized in that** spectral data from the NIR spectral range between 900 nm and 1600 nm are used to determine the amount of at least one powdery binder in a mixture with wood particles.

4. Method according to one of the preceding claims, **characterized in that** spectral data from the NIR spectral range between 1450 nm and 1600 nm, preferably between 1500 nm and 1600 nm, are used to determine the amount of at least one powdery binder in a mixture with wood particles.

5. Method according to one of claims 1-3, **characterized in that** spectral data from the NIR spectral range between 900 nm and 1100 nm, preferably between 900 nm and 1000 nm, are used to determine the amount of at least one powdery binder in a mixture with wood particles.

6. Method according to one of the preceding claims, **characterized in that** the quantitative amount of the powdery binder in the mixture with the wood particles is between 5 and 50% by weight, preferably between 7 and 40% by weight, more preferably between 10 and 30% by weight, even more preferably between 15 and 20% by weight (based on the total amount of the binder-wood particle mixture).

7. Process according to one of the preceding claims, **characterized in that** a naturally occurring binder is used as a biodegradable binder, in particular selected from a group comprising starch, cellulose derivatives, such as carboxymethyl cellulose, chitosan, gluten-containing binders, such as hide glue, bone glue, leather glue; milk-protein-containing binders, in particular from the group of caseins, and plant-protein-containing binders, in particular from the group of soy binders, agar-agar, alginate, gelatin, guar gum, gum arabic, xanthan gum, starch, pectins, locust bean gum, polysaccharides such as carrageenan.

8. Process according to one of claims 1-6, **characterized in that** a synthetic binder is used as biodegradable binder, in particular selected from the group comprising saponified polyvinyl alcohol, polycaprolactam-polyamide, polylactate, aliphatic polyester resins, in particular polybutylene succinate, polybutylene succinate-adipate, polyethylene-polypropylene composite resin, preferably polylactates.

9. Method according to one of the preceding claims, **characterized in that** wood chips, wood fibres and/or wood strands are used as wood particles.

10. Method according to one of the preceding claims, **characterized in that** the determination of the amount of at least one powdery binder in a mixture with wood particles is carried out continuously online in a production line for wood-based panels.

11. Method according to one of the preceding claims, **characterized in that** the determination of the amount of at least one powdery binder in a mixture with wood particles is carried out off-line.

12. Process for the production of wood-based panels comprising:
a) Production of wood particles from suitable woods,
b) Interim storage of the wood particles, if necessary, especially in silos or bunkers,
c) Drying the wood particles,
d) Sorting or sifting the wood particles according to the size of the wood strands,
e) Mixing the wood particles with at least one biodegradable powdery binder;
f) applying the mixture of wood particles and the at least one powdery binder to a conveyor belt by means of air and/or throw sifting, and
g) Pressing the mixture of wood particles and the at least one powdery binder arranged on the conveyor belt,
wherein the determination of the amount of the at least one powdery binder in the mixture with wood particles is carried out according to a method according to one of claims 1-11,
i) after mixing the wood particles with the at least one powdery binder but before applying the mixture of wood particles and the at least one powdery binder to a conveyor belt (after step e), and/or
ii) after the mixture of wood particles and the at least one powdery binder has been applied to a conveyor belt by means of air and/or throw sifting, but before the mixture of wood particles and the at least one powdery binder arranged on the conveyor belt has been pressed (according to step f), and/or
iii) After pressing the mixture of wood particles and the at least one powdery binder arranged on the conveyor belt into a wood particle cake (after step g).

## Revendications

1. Procédé de détermination de la quantité d'au moins un liant pulvérulent biodégradable dans un mélange avec des particules de bois,
comprenant les étapes
- de fourniture de mélanges composés d'au moins un liant pulvérulent et de particules de bois en tant qu'échantillons de référence, dans lequel l'au moins un liant pulvérulent et des particules de bois sont présents dans les mélanges selon des rapports de mélanges définis respectivement de manière quantitative,
- d'enregistrement d'au moins un spectre NIR des échantillons de référence en utilisant au moins une tête de mesure NIR dans une plage de longueurs d'onde entre 900 nm et 1700 nm,
- de fourniture d'un mélange composé d'au moins un liant pulvérulent et de particules de bois à mesurer,
- d'enregistrement d'au moins un spectre NIR du mélange composé d'au moins un liant pulvérulent avec des particules de bois en utilisant l'au moins une tête de mesure NIR dans une plage de longueurs d'onde entre 900 nm et 1700 nm, et
- de détermination de la proportion quantitative du liant pulvérulent dans le mélange composé du liant pulvérulent et de particules de bois par comparaison au spectre NIR enregistré pour les échantillons de référence.

2. Procédé selon la revendication 1, **caractérisé en ce que** des données spectrales issues de la plage spectrale globale enregistrée sont utilisées pour la détermination de la quantité d'au moins un liant pulvérulent dans un mélange avec des particules de bois.

3. Procédé selon l'une quelconque des revendications précédentes, **caractérisé en ce que** des données spectrales issues de la plage spectrale NIR entre 900 nm et 1600 nm sont utilisées pour la détermination de la quantité d'au moins un liant pulvérulent dans un mélange avec des particules de bois.

4. Procédé selon l'une quelconque des revendications précédentes, **caractérisé en ce que** des données spectrales issues de la plage spectrale NIR entre 1450 nm et 1600 nm, de manière préférée entre 1500 nm et 1600 nm, sont utilisées pour la détermination de la quantité d'au moins un liant pulvérulent dans un mélange avec des particules de bois.

5. Procédé selon l'une quelconque des revendications 1 - 3, **caractérisé en ce que** des données spectrales issues de la plage spectrale NIR entre 900 nm et 1100 nm, de manière préférée entre 900 nm et 1000 nm, sont utilisées pour la détermination de la quantité d'au moins un liant pulvérulent dans un mélange avec des particules de bois.

6. Procédé selon l'une quelconque des revendications précédentes, **caractérisé en ce que** la proportion de quantité du liant pulvérulent dans le mélange avec les particules de bois est comprise entre 5 et 50 % en poids, de manière préférée entre 7 et 40 % en poids, en particulier de manière préférée entre 10 et 30 % en poids, de manière davantage préférée entre 15 et 20 % en poids (par rapport à la quantité totale du mélange de liant et de particules de bois).

7. Procédé selon l'une quelconque des revendications précédentes, **caractérisé en ce qu'**un liant se formant naturellement est utilisé en tant que liant biodégradable, choisi en particulier parmi un groupe contenant de l'amidon, des dérivés de cellulose, comme de la cellulose de carboxyméthyle, du chitosan, des liants contenants du gluten, comme de la colle de peau, de la colle d'os, de la colle de cuir ; des liants contenant de protéines de lait, en particulier issus du groupe des caséines, et des liants contenant des protéines végétales, en particulier issus du groupe des liants à base de soja, de l'agar-agar, de l'alginate, des gélatines, de la gomme de guar, de la gomme arabique, de la gomme xanthane, de l'amidon, des pectines, de la gomme de caroube, des polysaccharides, comme de la carraghénane.

8. Procédé selon l'une quelconque des revendications 1 - 6, **caractérisé en ce qu'**un liant synthétique est utilisé en tant que liant biodégradable, choisi en particulier parmi le groupe contenant de l'alcool polyvinylique saponifié, du polyamide de polycaprolactame, du polylactate, des résines de polyester aliphatiques, en particulier du succinate de polybutylène, de l'adipate de succinate de polybutylène, de la résine composite de polyéthylène et de polypropylène, de manière préférée des polylactates.

9. Procédé selon l'une quelconque des revendications précédentes, **caractérisé en ce que** des copeaux de bois, des fibres de bois et/ou des panneaux à copeaux de bois sont utilisés en tant que particules de bois.

10. Procédé selon l'une quelconque des revendications précédentes, **caractérisé en ce que** la détermination de la quantité d'au moins un liant pulvérulent dans un mélange avec des particules de bois est effectuée en continu en ligne dans une ligne de production pour des panneaux en matériau dérivé du bois.

11. Procédé selon l'une quelconque des revendications précédentes, **caractérisé en ce que** la détermination de la quantité d'au moins un liant pulvérulent dans un mélange avec des particules de bois est effectuée hors ligne.

12. Procédé de fabrication de panneaux en matériau dérivé du bois comprenant :
a) la fabrication de particules de bois à partir de bois adaptés,
b) éventuellement l'entreposage temporaire des particules de bois en particulier dans des silos ou des abris,
c) le séchage des particules de bois,
d) le tri ou le criblage des particules de bois selon la taille des panneaux à copeaux de bois,
e) le mélange des particules de bois avec au moins un liant pulvérulent biodégradable ;
f) l'application du mélange composé de particules de bois et de l'au moins un liant pulvérulent sur une bande transporteuse au moyen d'un criblage à air et/ou par éjection, et
g) le compactage du mélange composé de particules de bois et de l'au moins un liant pulvérulent, disposé sur la bande transporteuse,
dans lequel la détermination de la quantité de l'au moins un liant pulvérulent dans le mélange avec des particules de bois selon un procédé selon l'une quelconque des revendications 1 - 11 est effectuée
i) après le mélange des particules de bois avec l'au moins un liant pulvérulent toutefois avant encore l'application du mélange composé de particules de bois et de l'au moins un liant pulvérulent sur une bande transporteuse (après l'étape e), et/ou
ii) après l'application du mélange composé de particules de bois et de l'au moins un liant pulvérulent, sur une bande transporteuse au moyen d'un criblage à air et/ou d'un criblage par éjection, toutefois avant encore le compactage du mélange composé de particules de bois et de l'au moins un liant pulvérulent, disposé sur la bande transporteuse (après l'étape f) et/ou
iii) après le compactage du mélange composé de particules de bois et de l'au moins un liant pulvérulent, disposé sur la bande transporteuse en un gâteau de particules de bois (après l'étape g).
